# EUROPÄISCHE PATENTSCHRIFT

(11) **EP 2 016 915 B1**
(45) Veröffentlichungstag und Bekanntmachung des Hinweises auf die Patenterteilung: **23.06.2010**
(21) Anmeldenummer: 07014237.7
(22) Anmeldetag: 20.07.2007
(51) Int. Cl.: A61B 17/70, A61F 2/44, A61B 17/02

(54) **Dornfortsatzimplantat**
Spinous process implant
Implant pour processus spinosus

(43) Veröffentlichungstag der Anmeldung: 21.01.2009
(73) Patentinhaber: Röbling, Christian, 79104 Freiburg (DE); Ackermann, Rolf, 78604 Rietheim-Weilheim (DE)
(72) Erfinder: Röbling, Christian, 79104 Freiburg (DE); Ackermann, Rolf, 78604 Rietheim-Weilheim (DE)
(74) Vertreter: Patentanwälte Westphal, Mussgnug & Partner

(56) Entgegenhaltungen:
- WO-A-02/07623
- WO-A-03/015645
- DE-A1-102005 005 694
- US-A1- 2003 216 736
- US-A1- 2005 245 937

## Beschreibung

Die Erfindung betrifft ein Dornfortsatzimplantat.

Die natürliche Degeneration der Wirbelsäule führt zu einer Verdickung der knöchernen Wirbelkörper und der Wirbelgelenke. Die Bandscheiben verlieren im Laufe der Zeit ihre Flexibilität und zeigen Vorwölbungen. Im Rückenmark gelegene Bänder verdicken sich. Die Folge dieser Degenerationen ist eine Verengung des Wirbelsäulenkanals, auch Spinalstenose genannt.

Durch den Wirbelsäulenkanal verlaufen unterhalb des Rückenmarks wichtige Nervenbahnen, die als typische Alterserscheinungen bei der lumbalen Spinalstenose zu Rückenschmerzen führen. Zur Behandlung der Spinalstenose ist es bekannt, ein Dornfortsatzimplantat zwischen zwei benachbarte Dornfortsätze zu implantieren, um den Abstand zwischen den beiden Dornfortsätzen zu vergrößern und auf diese Weise die Beschwerden zu lindern.

Einen Überblick über verschiedene Dornfortsatzimplantate gibt der Artikel P. Khoueir, K. A. Kim, M. Y. Wang: "Classification of posterior dynamic stabilization devices", Neurosurg. Focus, Vol. 22, Seite 1 ff. (Januar 2007).

Bekannt sind beispielsweise U-förmige Implantate, deren Schenkel an den einander zugewandten Seiten der Dornfortsätze anliegen und durch Federwirkung die Dornfortsätze auseinander drücken. Weiterhin bekannt sind auch verformbare Implantate, welche zunächst zusammengepresst werden, um sie zwischen die Dornfortsätze einzufügen, WO sie sich wieder ausdehnen, um die Dornfortsätze auseinander zu drücken.

Bekannt sind auch im Wesentlichen H-förmige Dornfortsatzimplantate mit einem Implantatgrundkörper, welcher zwischen zwei Dornfortsätzen zu liegen kommt, an welchem ein erstes und ein zweites Sicherungselement derart angeordnet sind, dass die Sicherungselemente auf verschiedenen Seiten der Dornfortsätze zu liegen kommen. Der Implantatgrundkörper ist dabei derart ausgeformt, dass er die Dornfortsätze auseinander drückt, während die Sicherungselemente beidseitig an den Dornfortsätzen anliegen, um die Position des Implantatgrundkörpers zu fixieren.

Beispielsweise ist der US 5 860 977 ein derartiges zweiteiliges Dornfortsatzimplantat zu entnehmen. Das erste Sicherungselemente ist an dem Implantatgrundkörper angeordnet. Dieses erste Teil muss durch einen ersten Schnitt seitlich der Dornfortsätze bzw. der Wirbelsäule zwischen die Dornfortsätze eingebracht werden, woraufhin durch einen zweiten Schnitt auf der anderen Seite der Dornfortsätze bzw. der Wirbelsäule das zweite Sicherungselement an dem Implantatgrundkörper im Körper des Patienten befestigt werden kann. Um ein derartiges bekanntes H-förmiges Dornfortsatzimplantat zu implantieren, sind somit zwei Schnitte von Nöten, was zu einem aufwändigen Operationseingriff führt. Zudem wird bei bekannten H-förmigen Dornfortsatzimplantaten das zweite Sicherungselement aufgeschraubt, was bei bereits in den Patienten eingebrachten Implantatgrundkörper für den Operateur sehr schwierig ist und die Operationsdauer verlängert. Zwar sind der US 5 860 977 auch aufschnappbare zweite Sicherungselemente zu entnehmen, welche jedoch weiterhin den Nachteil von zwei Schnitten auf verschiedenen Seiten der Dornfortsätze mit sich bringen.

Als weiterer Stand der Technik wird die US 2005/0245937 genannt.

Die Aufgabe der Erfindung besteht daher darin, ein Dornfortsatzimplantat bereitzustellen, welches einfacher zu implantieren ist.

Die Aufgabe der Erfindung wird gelöst durch ein Dornfortsatzimplantat mit den Merkmalen des Patentanspruchs 1.

Vorteilhafte Ausgestaltungen und Weiterbildungen der Erfindung sind in den Unteransprüchen angegeben.

Erfindungsgemäß sind sowohl das erste Sicherungselement als auch das zweite Sicherungselement an dem Implantatgrundkörper angeordnet, wobei das erste Sicherungselement relativ zu dem Implantatgrundkörper fixiert ist und der Implantatgrundkörper relativ zu dem zweiten Sicherungselement um eine Längsachse zwischen einer ersten Position und einer zweiten Position verdrehbar an dem zweiten Sicherungselement angeordnet ist. Weiterhin ist ein Arretiermechanismus zur Fixierung des Implantatgrundkörpers in der zweiten Position vorgesehen. Dieses Dornfortsatzimplantat ist derart ausgestaltet, dass es durch einen einzigen Schnitt auf einer Seite der Dornfortsätze zwischen die beiden benachbarten Dornfortsätze implantiert werden kann. Beim Einbringen zwischen die beiden Dornfortsätze befindet sich der Implantatgrundkörper zunächst in der ersten Position und kann durch den einzigen gesetzten Schnitt mit dem ersten Sicherungselement voran in den Zwischenraum zwischen den beiden Dornfortsätzen derart eingeschoben werden, dass das erste Sicherungselement durch den Zwischenraum zwischen den beiden Dornfortsätzen bis auf die andere Seite der Dornfortsätze geschoben werden kann. Das zweite Sicherungselement liegt anschließend seitlich an den Dornfortsätzen an, und zwar auf der Seite der Wirbelsäule, auf welcher der einzige Schnitt gesetzt wurde. In dieser Position kann nun der Implantatgrundkörper mit dem ersten Sicherungselement um seine Längsachse gedreht werden, so dass anschließend auch das erste Sicherungselement seitlich an den Dornfortsätzen zu liegen kommt und das Dornfortsatzimplantat nicht wieder zurückgezogen werden kann. In dieser gewünschten Position, d.h. der zweiten Position des Implantatgrundkörpers, erfolgt die Fixierung des Implantatgrundkörpers gegen Verdrehung des Implantatgrundkörpers gegen das zweite Sicherungselement durch den Arretiermechanismus. Durch das erfindungsgemäße Dornfortsatzimplantat wird es möglich, das Dornfortsatzimplantat lediglich mit einem einzigen Schnitt zu implantieren, so dass das Operationsverfahren deutlich weniger aufwändiger wird. Zudem ist kein aufwändiges Verschrauben des zweiten Sicherungselements an dem Implantatgrundkörper von Nöten, da das zweite Sicherungselement bereits vom Hersteller an dem Implantatgrundkörper angeordnet wird und der Operateur lediglich eine relative Drehung zwischen dem zweiten Sicherungselement und dem Implantatgrundkörper in die zweite Position vornehmen muss, um den Implantatgrundkörper in die gewünschte relative Lage zu dem zweiten Sicherungselement zu bringen. Der Arretiermechanismus bietet die Möglichkeit, nach Positionierung des Implantats und gewünschter Ausrichtung des Implantatgrundkörpers relativ zu dem zweiten Sicherungselement auf einfache Art und Weise diese Position zu fixieren.

Der Arretiermechanismus ist vorzugsweise ein Rastmechanismus, welcher besonders einfach zu arretieren ist und somit den Operationsaufwand und die möglichen Fehlerquellen auf ein Mindestmaß reduziert.

Erfindungsgemäß ist der Arretiermechanismus durch einen Splint gebildet. Dieser ist zwischen einer ersten Position und einer zweiten Position verschiebbar und wird von einer Rastwelle des Implantatgrundkörpers durchsetzt. Dabei weist die Rastwelle einen unrunden Abschnitt auf, welcher in der ersten Position des Splints um ihre Längsachse in einer Ausnehmung des Splints drehbar ist, wobei in der zweiten Position des Splints die Ausnehmung einen Formschluss mit dem unrunden Abschnitt der Rastwelle bildet, wodurch eine Drehung der Rastwelle verhindert wird. Auf diese Art und Weise wird ein besonders einfacher Arretiermechanismus gebildet, der insbesondere keine Fehlerquellen bietet, so dass das Implantieren einfacher und sicherer wird.

Der Splint ist vorzugsweise in einer Aufnahme in dem zweiten Sicherungselement geführt, um ihn gegen Verdrehung zu sichern.

Vorzugsweise ist an dem Splint wenigstens eine Rastnase angeordnet, welche in der zweiten Position des Implantatgrundkörpers mit einer Rastausnehmung in Eingriff bringbar ist. Auf diese Weise verhindert der Splint nicht nur eine Verdrehung des Implantatgrundkörpers aus der zweiten Position in die erste Position, sondern bildet zusätzlich einen Verriegelungsmechanismus, so dass auch der Splint in dieser Position arretiert und nicht versehentlich wieder gelöst werden kann, was eine Drehung des Implantatgrundkörpers ermöglichen würde.

Vorzugsweise ist an dem Implantatgrundkörper ein Vorsprung angeordnet ist, welcher mit zwei an dem zweiten Sicherungselementen angeordneten Vorsprüngen derart zusammenwirkt, dass die Drehbewegung des Implantatgrundkörpers relativ zu dem zweiten Sicherungselement auf einen bestimmten Winkelbereich begrenzt wird. Selbstverständlich ist es auch möglich, dass nur ein Vorsprung an dem zweiten Sicherungselement und entsprechend zwei im Winkelabstand zueinander angeordnete Vorsprünge an dem Implantatgrundkörper angeordnet sind, welche dementsprechend zusammenwirken.

Vorzugsweise ist der an dem Implantatgrundkörper angeordnete Vorsprung an wenigstens einem der an dem zweiten Sicherungselement angeordneten Vorsprünge über einen Rastmechanismus fixierbar ist, welcher vorzugsweise den Arretiermechanismus bildet, da auf diese Weise ein einfacher Arretiermechanismus gebildet wird, denn sobald die Endposition der Drehung erreicht wird und der an dem Implantatgrundkörper angeordnete Vorsprung an einen der an dem zweiten Sicherungselement angeordneten Vorsprünge anschlägt, greift der Rastmechanismus ein und verhindert ein Zurückdrehen des Implantatgrundkörpers gegen das zweite Sicherungselement.

Vorzugsweise sind der an dem Implantatgrundkörper angeordnete Vorsprung als Rastelement und die an dem Sicherungselement angeordneten Vorsprünge als Zapfen ausgebildet, da in dieser Ausbildungsform ein Rastmechanismus als einfaches Aufschnappen auf die im wesentlichen zylindrischen Zapfen realisiert werden kann.

Bei einer besonders bevorzugten Ausführungsform der Erfindung ist an dem Implantatgrundkörper ein in Längsrichtung vorstehender, exzentrisch angeordneter Zapfen angeordnet, welcher in eine kreisbogenförmige Nut des zweiten Sicherungselements eingreift. Selbstverständlich ist es auch möglich, dass der Zapfen an dem zweiten Sicherungselement und die kreisbogenförmige Nut an dem Implantatgrundkörper angeordnet ist. Die Nut und der darin bei Drehung des Implantatgrundkörpers gegen das Sicherungselement laufende Zapfen verhindern, dass der Implantatgrundkörper beliebig um seine Längsrichtung gedreht werden kann. Je nach Länge der Nut, wobei die Nut vorzugsweise einen Winkelbereich von etwa 90° überstreicht, ist die Drehbewegung des Implantatgrundkörpers gegen das zweite Sicherungselement begrenzt. Die beiden Enden der Nut bilden Anschläge für die Drehbewegung des Implantatgrundkörpers, wobei durch Anschlag des Zapfens an die beiden Enden der Nut jeweils die erste und die zweite Position des Implantatgrundkörpers relativ zum Sicherungselement definiert werden. Durch eine derartige Nut ist gewährleistet, dass keinesfalls der Implantatgrundkörper zu weit gedreht werden kann, wodurch Beschädigungen des umliegenden Gewebes im Patienten vermieden werden, wobei weiterhin diese Vorgaben die Operation dahingehend vereinfachen, dass der Operateur automatisch den Implantatgrundkörper in die richtige Richtung und die richtige zweite Position dreht. Je nachdem auf welcher Seite der Dornfortsätze der Schnitt zur Operation gesetzt werden soll, und in welche Richtung die Drehung des Implantatgrundkörpers gegen das zweite Sicherungselement erfolgen soll, muss die kreisbogenförmige Nut an entsprechender Stelle angeordnet werden. Alternativ zu einer Nut können auch zwei Vorsprünge verwendet werden, die jeweils einen Anschlag für einen Zapfen oder einen beliebig geformten Vorsprung bei der relativen Drehbewegung des Implantatgrundkörpers gegen das zweite Sicherungselement bilden.

Besonders bevorzugt ist das erste Sicherungselement einstückig mit dem Implantatgrundkörper zu einem Spreizelement verbunden. Dadurch wird es insbesondere möglich, das Spreizelement derart auszuformen, dass es eine anatomisch günstige Formgebung aufweist. Zudem ist die Fertigung eines einzelnen Spreizelements kostengünstiger als die Fertigung von einem separaten ersten Sicherungselement und einem separaten Implantatgrundkörper, welche anschließend relativ zueinander fixiert werden müssen.

Besonders bevorzugt ist das Spreizelement aus einem Kunststoff oder aus einem biokompatiblen Material mit hohem Elastizitätsmodul, insbesondere aus Polyetheretherketon (PEEK), gefertigt, da dieses Material einfach zu verarbeiten und biokompatibel ist.

Das Spreizelement weist vorzugsweise eine anatomisch geformte Anlagefläche auf, welche insbesondere zur Anlage an die benachbarten Dornfortsätze geeignet ist, um eine sichere Fixierung des Implantatgrundkörpers zwischen den beiden Dornfortsätzen zu gewährleisten.

Bei einer besonders bevorzugten Ausführungsform weist das erste Sicherungselement eine anatomisch geformte Spitze zur Durchführung des ersten Sicherungselements durch den Zwischenraum zwischen den Dornfortsätzen auf. Auf diese Weise wird sichergestellt, dass das umliegende Gewebe möglichst wenig beschädigt wird und das Implantat einfach und sicher in den Zwischenraum zwischen den Dornfortsätzen eingeführt werden kann.

Gemäß einer besonders bevorzugten Ausführungsform der Erfindung weist das zweite Sicherungselement eine anatomisch geformte Anlagefläche auf, welche insbesondere zur Anlage an dem Lamina und an dem Fassettengelenk geeignet ist. Das zweite Sicherungselement liegt damit nach Einführung des Implantats nicht nur seitlich an den Dornfortsätzen an, sondern ist in dieser Position auch derart stabilisiert, dass die Anlagefläche im Wesentlichen parallel zur Wirbelsäule anliegt, so dass eine Verdrehsicherung des zweiten Sicherungselements um die Längsachse des Implantats gegeben ist und der Implantatgrundkörper beispielsweise mit Hilfe eines speziellen Instruments gegen das zweite Sicherungselement gedreht werden kann, ohne dass eine zusätzliche Fixierung des zweiten Sicherungselements in dieser Position gegen Verdrehung von Nöten ist.

Vorzugsweise weist das zweite Sicherungselement wenigstens ein, vorzugsweise zwei bis vier Schwenkelemente auf, welche relativ zu dem zweiten Sicherungselement bewegbar, insbesondere schwenkbar sind, insbesondere zwischen einer eingefahrenen Position und einer ausgefahrenen Position. Dabei befinden sich bevorzugt die Schwenkelemente in der eingefahrenen Position, wenn sich der Implantatgrundkörper in der ersten Position befindet, und in der ausgefahrenen Position, wenn sich der Implantatgrundkörper in der zweiten Position befindet. Ein Einfahren der Schwenkelemente bewirkt eine Verkleinerung der äußeren Dimensionen des zweiten Sicherungselements. Die Kopplung der Bewegungen der Schwenkelemente und des Implantatgrundkörpers vereinfacht das Implantieren wesentlich.

Vorzugsweise weist das Implantat eine Längsbohrung mit einem Einstich auf, wobei der Einstich bewirkt, dass das Implantat insbesondere im Bereich des Implantatgrundkörpers eine gewisse Flexibilität aufweist.

Vorteilhafterweise ist das Implantat kannuliert, um einen Draht oder ähnliches durch das Implantat durchführen zu können, welcher als Einführhilfe dient.

Bevorzugt ist in dem ersten Sicherungselement ausgehend etwa von der Längsachse eine Nut angeordnet. Insbesondere wenn durch das Implantat eine Einführhilfe gezogen wird, ist diese Nut von Vorteil, um die Einführhilfe eng an dem Implantat anliegend führen zu können.

Gemäß einer besonders bevorzugten Ausführungsform der Erfindung weist der Implantatgrundkörper einen im Wesentlichen ovalen oder elliptischen Querschnitt mit einer Hauptachse und einer Nebenachse auf. Dabei ist der ovale Querschnitt vorzugsweise derart orientiert, dass bei Einführen des Dornfortsatzimplantats mit dem Implantatgrundkörper in der ersten Position die Hauptachse parallel zur Längsrichtung der Dornfortsätze und die Nebenachse parallel zur Wirbelsäule verläuft, so dass bei Einführen des Implantatgrundkörpers in den Zwischenraum zwischen zwei Dornfortsätzen die Dornfortsätze kaum auseinandergespreizt werden und somit zunächst keine Verletzungsgefahr oder Beschädigungsgefahr besteht. Erst wenn der Implantatgrundkörper nach Einführen des Dornfortsatzimplantats in den Zwischenraum zwischen zwei Dornfortsätze in die zweite Position gedreht wird, kommt die Hauptachse parallel zur Wirbelsäule und die Nebenachse parallel zur Längsrichtung der Dornfortsätze zu liegen, so dass bei dieser Drehbewegung die beiden Dornfortsätze auseinandergedrückt werden.

Vorzugsweise ist dabei die Hauptachse etwa 2 mm länger als die Nebenachse, was bereits ausreichend ist, um die Dornfortsätze derart auseinander zu drücken, dass eine Schmerzlinderung beim Patienten erfolgt.

Die Erfindung wird anhand der nachfolgenden Figuren ausführlich erläutert. Es zeigt:
- Figur 1: eine Explosionsdarstellung eines Ausführungs- beispiels eines Dvrnfortsatzimplantats,
- Figur 2: eine perspektivische Darstellung eines zweiten Sicherungselements des Dornfortsatzimplantats gemäß Figur 1,
- Figur 3: eine perspektivische Darstellung einer Rastwelle des Dornfortsatzimplantats gemäß Figur 1,
- Figur 4: eine Seitenansicht der Rastwelle gemäß Figur 3,
- Figur 5: eine Seitenansicht der Rastwelle gemäß Figur 3 mit einer eingesetzten Schraube,
- Figur 6: eine Seitenansicht des zweiten Sicherungsele- ments gemäß Figur 2 mit eingesetzter Rastwelle gemäß Figur 5,
- Figur 7: eine perspektivische Darstellung der Rastwelle gemäß Figur 3 mit einem Splint 60 in einer ersten Position,
- Figur 8: eine perspektivische Ansicht der Rastwelle mit dem Splint gemäß Figur 7 mit dem Splint in einer zweiten Position,
- Figur 9: eine schematische Darstellung der Lage des Dornfortsatzimplantats im Körper eines Patienten,
- Figur 10: einen Querschnitt durch das Dornfortsatz- implantat gemäß Figur 1 entlang der Linie A-A,
- Figur 11: eine Draufsicht auf ein zweites Ausführungsbei- spiel eines zweiten Sicherungselements eines Dornfortsatzimplantats in einer ersten Position,
- Figur 12: das zweite Sicherungselement gemäß Figur 11 in einer zweiten Position,
- Figur 13: ein Längsschitt durch das Dornfortsatzimplantat gemäß Figur 11 und
- Figur 14: eine Draufsicht auf das erste Sicherungselement des Dornfortsatzimplantats gemäß Figur 11.

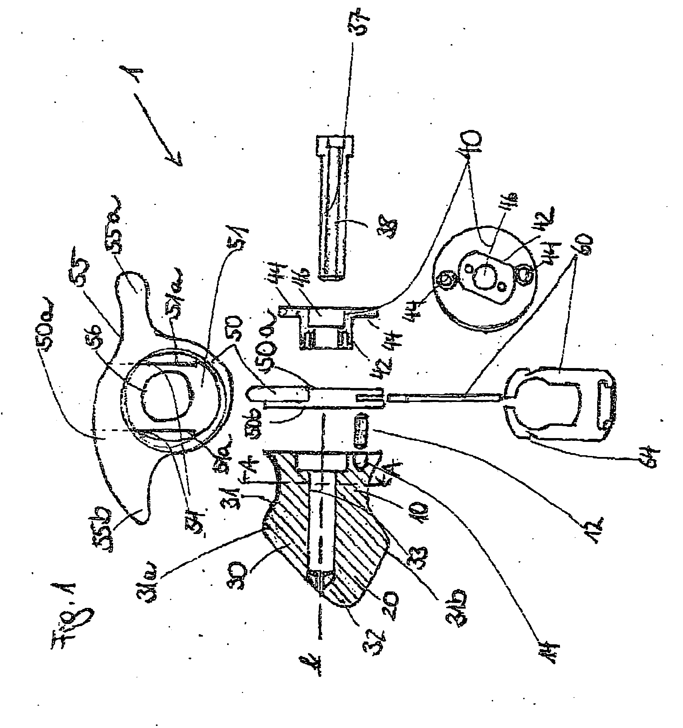

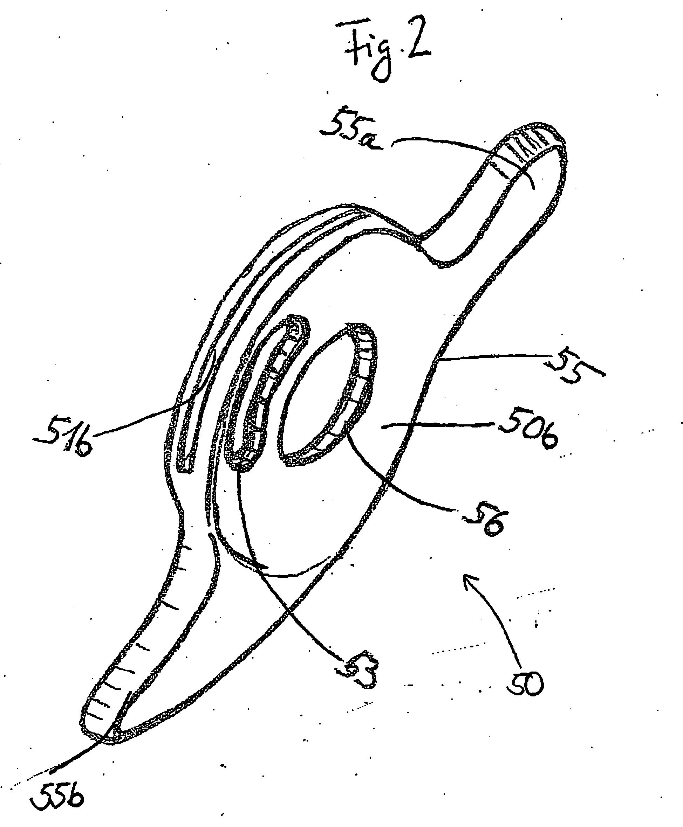

In den Figuren 1 bis 10 sind verschiedene Ansichten, teilweise auch nur von verschiedenen Bestandteilen, eines Dornfortsatzimplantats 1 dargestellt, wobei gleiche Bezugsziffern stets gleiche Teile bezeichnen und der besseren Übersicht halber nicht in sämtlichen Figuren sämtliche Bezugszeichen aufgeführt sind.

Das Dornfortsatzimplantat 1 weist einen Implantatgrundkörper 10 und ein daran angeordnetes erstes Sicherungselement 20 auf, wobei der Implantatgrundkörper 10 und das erste Sicherungselement 20 einstückig als ein Spreizelement 30 mit einer Längsachse 1 ausgebildet sind. Durch das Spreizelement 30 verläuft eine Längsbohrung 33 im Wesentlichen koaxial zur Längsachse 1 des Spreizelements 30 bzw. des Dornfortsatzimplantats 1. Die Längsbohrung 33 weist in dem dem ersten Sicherungselement 20 abgewandten Ende einen erweiterten Bereich auf, in welchem eine Rastwelle 40 mit Hilfe einer Schraube 37 befestigt ist. Die Schraube 37 wird dabei durch eine Längsbohrung 46 der Rastwelle 40 geführt und in der Längsbohrung 33 des Spreizelements 30 verschraubt. Die Rastwelle 40 ist damit unverdrehbar gegenüber dem Spreizelement 30 angeordnet. Die Rastwelle 40 durchsetzt ein zweites Sicherungselement 50 in einer Öffnung 56 des zweiten Sicherungselements 50, wobei das zweite Sicherungselement 50 verdrehbar gegenüber der Rastwelle 40 angeordnet ist.

Die Rastwelle 40 weist auf ihrer dem Implantatgrundkörper 10 abgewandten Seite zwei Eingriffsbohrungen 44 auf, in welche ein spezielles Einsetzinstrument gesetzt werden kann.

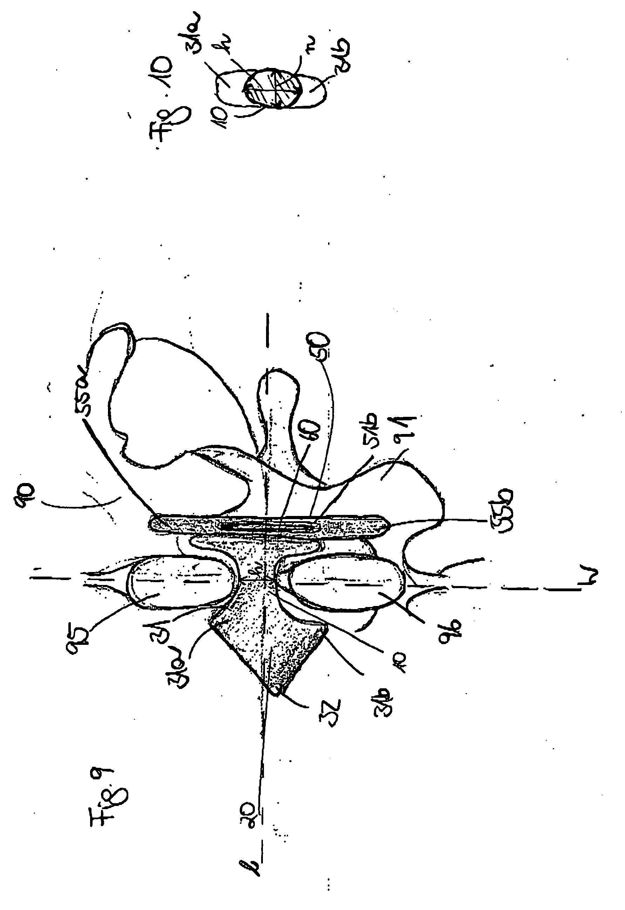

In der in Figur 9 dargestellten fertig implantierten Position, die im folgenden auch als zweite Position des Implantatgrundkörpers 10 relativ zum zweiten Sicherungselement 50 bezeichnet wird, liegt der Implantatgrundkörper 10 zwischen zwei Dornfortsätzen 95, 96 zweier benachbarter Wirbelkörper 90, 91, während das erste Sicherungselement 20 in Figur 9 links der Achse W der Wirbelsäule des Patienten und das zweite Sicherungselement 50 auf der anderen Seite der Dornfortsätze 95, 96 bzw. der Wirbelsäule, d. h. rechts im Bild in Figur 9, zu liegen kommt. Dadurch, dass die Sicherungselement 20, 50 in dieser Position in Richtung der Wirbelsäule breiter sind als der Zwischenraum zwischen den Dornfortsätzen 95, 96, wird das Dornfortsatzimplantat 1 gegen Verschiebungen entlang der Längsrichtung 1 des Dornfortsatzimplantats 1 gesichert. Dazu weist das Spreizelement 30 bzw. das erste Sicherungselement 20 eine Anlagefläche 31 mit Vorsprüngen 31a, 31b auf, wobei die Anlagefläche 31 derart ausgestaltet ist, dass sie sich aus dem Bereich zwischen den beiden Dornfortsätzen 95, 96 auf die Außenseite der Dornfortsätze 95, 96 erstreckt und die Vorsprünge 31a, 31b seitlich an den Dornfortsätzen 95, 96 anliegen. Auch das zweite Sicherungselement 50 weist zwei Vorsprünge 55a, 55b auf, welche verhindern, dass das Dornfortsatzimplantat 1 entlang der Längsrichtung 1 durch den Raum zwischen den beiden Dornfortsätzen 95, 96 bewegt werden kann.

Um das Dornfortsatzimplantat 1 in den Körper des Patienten implantieren zu können, muss zunächst ein Schnitt seitlich von der Achse W der Wirbelsäule gesetzt werden. Vorliegend wird der Schnitt in Figur 9 seitlich nach rechts versetzt parallel zur Achse W der Wirbelsäule gesetzt. Das Dornfortsatzimplantat 1 wird als ein Implantat mit Implantatgrundkörper 10, dem ersten Sicherungselement 20 und dem zweiten Sicherungselement 50 als ein Teil implantiert. Dazu befindet sich zunächst der Implantatgrundkörper 10 mit dem ersten Sicherungselement 20 und somit der Spreizkegel 30 in einer ersten Position relativ zu dem zweiten Sicherungselement 50. An dem Implantatgrundkörper 10 ist dazu parallel zur Längsachse 1 ein Zapfen 12 in einer Sackbohrung 14 des Implantatgrundkörpers 10 angeordnet, welcher in Richtung auf das zweite Sicherungselement 50 aus dem Implantatgrundkörper 10 hervorragt. Das zweite Sicherungselement 50 weist eine dem Implantatgrundkörper 10 zugewandte Seite 50b und eine dem Implantatgrundkörper 10 abgewandte Seite 50a auf, wobei in der dem Implantatgrundkörper zugewandten Seite 50b eine im Wesentlichen kreisbogenförmige Nut 53 angeordnet ist, in welche der an dem Implantatgrundkörper 10 angeordnete Zapfen 12 eingreift und in welcher er bei Drehung des Implantatgrundkörpers 10 relativ zu dem zweiten Sicherungselement 50 entlang der Nut 53 bewegt wird. Die Nut 53 überstreicht im Wesentlichen einen Winkelbereich von 90°, wobei die Enden der kreisbogenförmigen Nut 53 zwei Anschläge für den Zapfen 12 bilden, so dass die Drehbewegung des Implantatgrundkörpers 10 gegenüber dem zweiten Sicherungselement 50 begrenzt wird. Befindet sich der Zapfen 12 an den Enden der kreisbogenförmigen Nut 53, so dass die Drehbewegung gestoppt wird, werden an einem Ende der kreisbogenförmigen Nut 53 die erste Position des Implantatgrundkörpers 10 relativ zum zweiten Sicherungselement 50 und an dem gegenüberliegenden Ende der kreisbogenförmigen Nut 53 die zweite Position des Implantatgrundkörpers 10 relativ zu dem zweiten Sicherungselement 50 definiert.

Beim Implantieren des Dornfortsatzimplantats 1 in den Körper des Patienten befindet sich der Implantatgrundkörper 10 und somit das Spreizelement 30 zunächst in der ersten Position relativ zu dem zweiten Sicherungselement 50, so dass der Zapfen 12 an einem Ende der kreisbogenförmigen Nut 53 anschlägt. In dieser ersten Position wird das Dornfortsatzimplantat 1 derart durch den Schnitt seitlich der Achse W der Wirbelsäule eingeführt, dass die Vorsprünge 55a, 55b des zweiten Sicherungselements 50 parallel zur Achse W der Wirbelsäule verlaufen, während jedoch die Vorsprünge 31a, 31b mit ihrer Verbindungslinie senkrecht zur Achse W der Wirbelsäulen verlaufen. Im Bereich zwischen den Dornfortsätzen 95, 96 wurde das Ligamentum Interspinosum in Faserrichtung gespalten, so dass es möglich ist, das Spreizelement 30 zwischen den beiden Dornfortsätzen 95, 96 auf die gegenüberliegende Seite der Achse W der Wirbelsäulen 40 hindurchzuschieben. Das Spreizelement 30 weist dazu an dem dem zweiten Sicherungselement 50 abgewandten Ende eine anatomisch ausgeformte Spitze 32 auf, die das Durchschieben des Spreizelements 30 durch den Zwischenraum zwischen den beiden Dornfortsätzen 95, 96 erleichtert. Das Dornfortsatzimplantat 1 wird soweit eingeschoben, bis der Implantatgrundkörper 10 zwischen den beiden Dornfortsätzen 95, 96 zu liegen kommt.

Das zweite Sicherungselement 50 ist nun derart positioniert, dass die Vorsprünge 55a, 55b verhindern, dass das Dornfortsatzimplantat 1 weiter in den Bereich zwischen den Dornfortsätzen 95, 96 geschoben werden kann. Die Vorsprünge 55a, 55b sind Teil einer Anlagefläche 55, mit welcher das zweite Sicherungselement 50 an dem Lamina einerseits und dem Fassettengelenk andererseits anliegt. Der Vorsprung 55a bildet die caudale Anlagefläche, der Vorsprung 55b die craniale Anlagefläche. Dabei ist der Vorsprung 55a etwa löffelförmig und der Vorsprung 55b etwa dreieckig zulaufend ausgebildet. Die Vorsprünge 55a, 55b sind insbesondere derart ausgeformt, dass auch in zwei direkt an einem Wirbelkörper caudal und cranial angrenzende Zwischenräume zwischen den benachbarten Dornfortsätzen jeweils ein Dornfortsatzimplantat 1 implantiert werden kann, ohne sich gegenseitig zu behindern. Da die Vorsprünge 55a, 55b gleichzeitig an knöchernen Strukturen anliegen, bewirkt diese Anlage eine Verdrehsicherung des zweiten Sicherungselements 50, so dass ohne weitere Sicherungsmaßnahmen der Implantatgrundkörper 10 mit Hilfe des Einsetzinstruments, welches in die Eingriffsbohrungen 44 der Rastwelle 40 eingesetzt wird, gegenüber dem zweiten Sicherungselement 50 gedreht werden kann. Es erfolgt eine Drehung um etwa 90°, bis der Zapfen 12 des Implantatgrundkörpers 10 an das andere Ende der kreisbogenförmigen Nut 53 des zweiten Sicherungselements 50 anschlägt. In dieser zweiten Position liegen nun auch die Vorsprünge 31a, 31b des Spreizelements 30 derart, dass ihre Verbindungslinie im Wesentlichen parallel zur Achse W der Wirbelsäule wie in Figur 9 gezeigt liegen.

Der Implantatgrundkörper 10 weist einen im Wesentlichen ovalen Querschnitt auf, wie in Figur 10 gezeigt. Der Querschnitt, welcher auch etwa elliptisch ausgebildet ist, weist eine Hauptachse h und eine Nebenachse n auf. Die Hauptachse h ist dabei länger als die Nebenachse n, insbesondere um einige Millimeter. Der Querschnitt des Implantatgrundkörpers 10 ist so orientiert, dass in der ersten Position während des Implantierens die Nebenachse n parallel zur Achse W der Wirbelsäule verläuft, so dass die Breite des Implantatgrundkörpers 10 geringer ist und der Implantatgrundkörper 10 und das Spreizelement 30 einfacher in den Bereich zwischen den Dornfortsätzen 95, 96 eingeführt werden können. Bei Drehung des Implantatgrundkörpers 10 um 90° gegenüber dem zweiten Sicherungselement 50 nach Einbringen des Implantatgrundkörpers zwischen die benachbarten Dornfortsätze 95, 96 wird der Implantatgrundkörper 10 so gedreht, dass anschließend die Hauptachse h im Wesentlichen parallel zur Achse W der Wirbelsäule verläuft, so dass bei Drehung des Implantatgrundkörpers 10 um 90° gegenüber dem zweiten Sicherungselement 50 ein Aufspreizen erfolgt und die Dornfortsätze 95, 96 auseinander gespreizt werden. Im Gegensatz zu sonst üblichen Implantationsverfahren, bei welchen das Implantat bereits mit der gewünschten endgültigen Breite in den Bereich zwischen den Dornfortsätzen eingetrieben wird, besteht in diesem Fall weniger Verletzungsgefahr und das Einbringen des Dornfortsatzimplantats 1 gemäß der Erfindung ist deutlich vereinfacht. Insbesondere ist die Länge der Nebenachse n so bemessen, dass sie im Wesentlichen dem Abstand zwischen den Dornfortsätzen 95, 96 entspricht, während die Hauptachse h etwa 2 bis 4 mm länger als die Nebenachse n ist. Beispielsweise kann die Länge der Nebenachse n 8 mm betragen, während die Länge der Hauptachse h etwa 10 mm beträgt. Wesentlich bei dem vorliegenden Dornfortsatzimplantat 1 ist, dass dieses in einem Teil durch einen einzigen Schnitt seitlich der Wirbelsäule implantiert werden kann und nach Einführen des Dornfortsatzimplantats 1 lediglich eine Drehung um 90° zwischen dem Implantatgrundkörper 10 und dem zweiten Sicherungselement 50 vorgenommen werden muss, um das Dornfortsatzimplantat 1 richtig zu positionieren. Da das zweite Sicherungselement 50 bereits herstellerseitig an dem Implantatgrundkörper 10 montiert wurde, ist kein aufwändiges nachträgliches Anbringen im Körper des Patienten notwendig. Die Verdrehbarkeit zwischen dem Implantatgrundkörper 10 und dem zweiten Sicherungselement 50 ermöglicht es dabei, das Dornfortsatzimplantat 1 besonders einfach in den Körper des Patienten einzubringen und richtig zu positionieren.

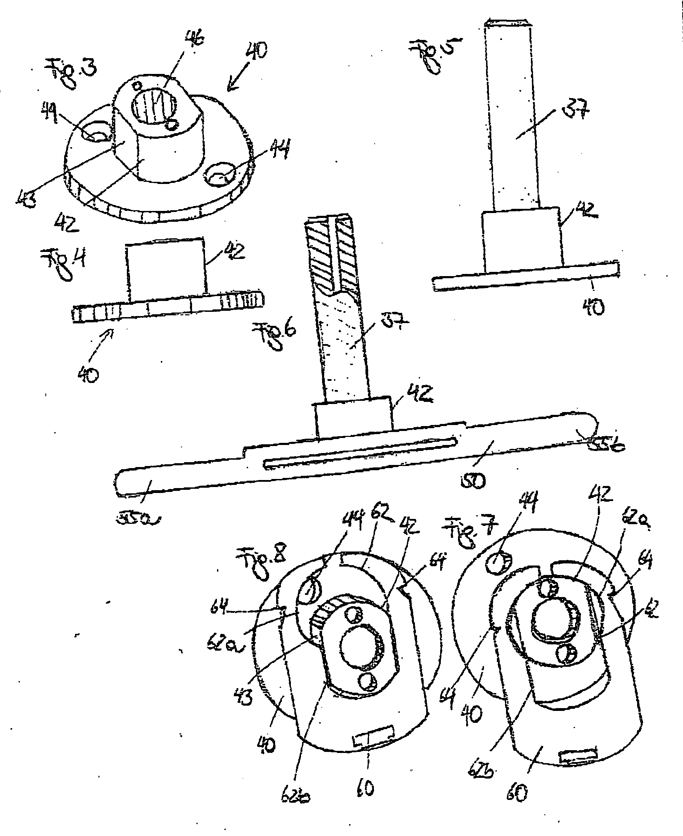

Um den Implantatgrundkörper 10 in der zweiten Position relativ zu dem zweiten Sicherungselement 50 zu arretieren, ist ein Arretiermechanismus vorgesehen. Die Rastwelle 40 weist dazu einen unrunden Abschnitt 42 auf, welcher im Wesentlichen zylindrisch ausgebildet ist, wobei der unrunde Abschnitt 42 einen Querschnitt derart aufweist, dass von einem im Wesentlichen kreisförmigen Querschnitt durch zwei parallele Schnitte zwei Kreiskappen entfernt wurden. Dadurch werden zwei parallel zueinander verlaufende Anlageflächen 43, wie beispielsweise in Figur 3 ersichtlich, gebildet.

In dem zweiten Sicherungselement 50 ist in einer Aufnahme 51 ein Splint 60 angeordnet. Der Splint 60 wird dabei in einer Ausnehmung 62 von dem unrunden Abschnitt 42 der Rastwelle 40 durchsetzt. Die Ausnehmung 62 weist einen ersten Bereich 62a und einen zweiten Bereich 62b auf, wobei der erste Bereich 62a in etwa kreisförmig ausgebildet ist, so dass die Rastwelle 40, wenn sie den Splint 60 in dem ersten Bereich 62a der Ausnehmung 62 durchsetzt, ungehindert rotieren kann. Der zweite Bereich 62b weist zwei parallel zueinander verlaufende Kanten auf, deren Abstand im Wesentlichen dem Abstand der beiden Anlageflächen 43 der Rastwelle 40 entspricht. Wird der Splint 60 aus einer ersten Position, in welcher die Rastwelle 40 die Ausnehmung 62 in dem ersten Bereich 62a durchsetzt, in eine zweite Position verschoben, in welcher die Rastwelle 40 die Ausnehmung 62 des Splints 60 in dem zweiten Bereich 62b durchsetzt, kommen die parallel verlaufenden Kanten des zweiten Bereichs 62b an den Anlageflächen 63 zu liegen und bilden somit einen Formschluss, welcher verhindert, dass die Rastwelle 40 gegenüber dem Splint 60 verdreht werden kann. Der Splint 60 ist in der Aufnahme 51 des zweiten Sicherungselements 50 unverdrehbar durch zwei parallel verlaufende Führungen 51b gesichert, so dass das Überführen des Splints 60 in die zweite Position einen Arretiermechanismus darstellt, welcher verhindert, dass das mit der Rastwelle 40 unverdrehbar verbundene Spreizelement 30 relativ zu dem in dem zweiten Sicherungselement 50 verdrehbar angeordneten Splint 60 verdreht werden kann. Der Splint 60 wird durch einen seitlichen Schlitz 51b in die Aufnahme 51 des zweiten Sicherungselements 50 eingeschoben, wobei er in der ersten Position seitlich über das zweite Sicherungselement hinausragt und in der zweiten Position bündig mit der Seitenfläche des zweiten Sicherungselements 50 abschließt. Wesentlich für den Arretiermechanismus ist, dass er einfach zu betätigen und sicher zu handhaben ist, wie es beispielsweise bei Formschluss oder auch bei einem Rastmechanismus der Fall ist. Eine Blockierung der Drehbewegung wird beispielsweise auch erzielt, wenn in der zweiten Position ein an dem zweiten Sicherungselement 50 angeordnetes Arretierelement in Richtung der Längsachse 1 derart verschoben wird, dass es in eine entsprechend geformte Ausnehmung an dem Implantatgrundkörper 10 eingreift.

Um zu verhindern, dass das Splint 60 versehentlich aus der zweiten Position wieder in die erste Position verschoben wird, sind an dem Splint 60 zwei Rastnasen 64 angeordnet, welche in der zweiten Position des Splints 60 in zwei Rastausnehmungen 54 in der Aufnahme 51 des zweiten Sicherungselements 50 einrasten.

Die Längsbohrung 33 des Spreizelements 30 weist einen Einstich auf, was zu einer höheren Flexibilität im Bereich zwischen den beiden Dornfortsätzen 95, 96 des Spreizelements 30 führt.

Die Schraube 37 ist kannuliert und weist dazu eine Bohrung 38 auf, durch welche ein Führungsdraht durch das Dornfortsatzimplantat 1 gezogen werden kann, um die Positionierung des komplett montierten Implantats zu erleichtern. Mit dem erfindungsgemäßen Dornfortsatzimplantat 1 ist somit ein einfaches Operationsverfahren möglich, in welchem lediglich ein einziger Schnitt seitlich versetzt zur Wirbelsäule vorgenommen werden muss, das Dornfortsatzimplantat 1 in einem Teil durch diesen Schnitt in der ersten Position des Implantatgrundkörpers 10 relativ zum zweiten Sicherungselement 50 implantiert werden kann, anschließend der Implantatgrundkörper 10 gegenüber dem zweiten Sicherungselement 50 verdreht wird, vorzugsweise um 90°, und anschließend der Implantatgrundkörper 10 in dieser zweiten Position arretiert wird, was vorzugsweise mit Hilfe des Splints 60 über einen Formschluss erfolgt. Bei der Verdrehung des Implantatgrundkörpers 10 gegen das zweite Sicherungselement 50 stützt sich das Sicherungselement 50 vorzugsweise an den knöchernen Strukturen der Wirbelsäule, insbesondere über die anatomisch geformte Anlagefläche 55 ab, um eine Verdrehsicherung zu bilden.

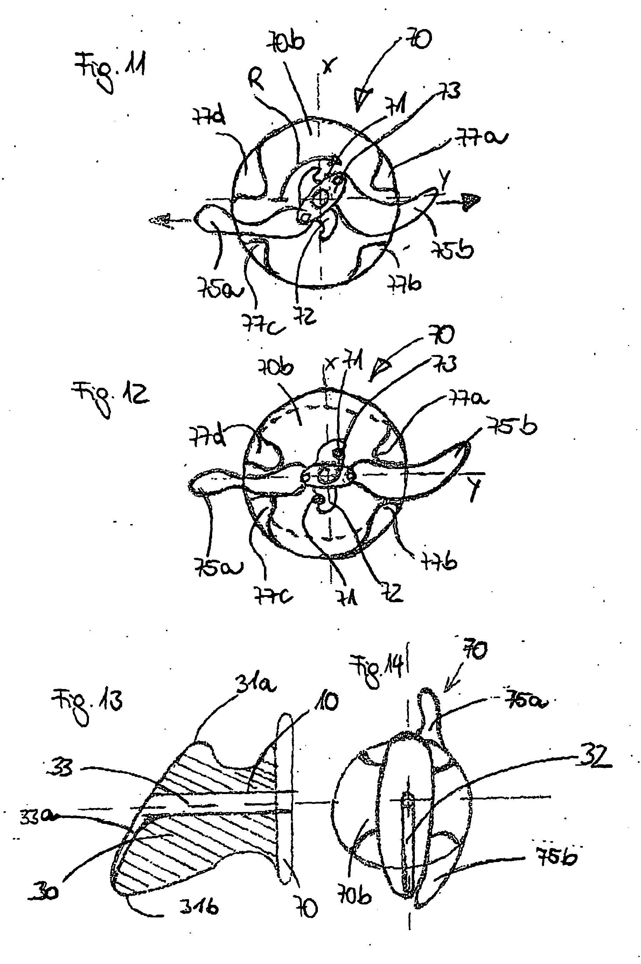

In den Figuren 11 bis 14 sind verschiedene Ansichten eines zweiten Ausführungsbeispiels dargestellt, wobei gleiche Bezugsziffern gleiche Teile auch des ersten Ausführungsbeispiels bezeichnen und der besseren Übersicht halber nicht in sämtlichen Figuren sämtliche Bezugszeichen aufgeführt sind. Das Dornfortsatzimplantat 1 gemäß dem zweiten Ausführungsbeispiel weist ein zweites Sicherungselement 70 auf, welches sich von dem zweiten Sicherungselement 50 des ersten Ausführungsbeispiels unterscheidet.

Das zweite Sicherungselement 70 weist eine dem Implantatgrundkörper 10. zugewandte Seite 70b auf, auf welche eine Draufsicht in den Figuren 11 und 12 zu sehen ist. Das zweite Sicherungselement 70 ist als im wesentlichen runde Scheibe ausgebildet, welche eine zentrale Öffnung 76 aufweist, durch welche das zweite Sicherungselement mit einem geeigneten nicht dargestellten Befestigungsmechanismus drehbar an dem Implantatgrundkörper 10 angeordnet werden kann. Zwischen dem Implantatgrundkörper 10 und dem zweiten Sicherungselement 70 ist ein Befestigungselement 73 angeordnet, welches drehfest mit dem Implantatgrundkörper 10 verbindbar ist. Das Befestigungselement 73 ist langgestreckt, beispielsweise im wesentlichen elliptisch ausgebildet, wobei an den beiden Enden jeweils ein Schwenkelement 75a, 75b in einer Ebene parallel zur Ebene des zweiten Sicherungselements 70 drehbar um eine Achse parallel zur Längsachse 1 gelagert angeordnet ist.

Bei Einführen des Implantats 1 ist das Befestigungselement 73 zunächst derart ausgerichtet, dass seine Längsachse im wesentlichen parallel zu der in den Figuren 11 und 12 eingezeichneten x-Achse und insbesondere auch im wesentlichen parallel zu der Hauptachse h des Spreizelements 30 des Implantatgrundkörpers 10 verläuft. Die beiden Schwenkelemente 75a, 75b befinden sich dabei in einer eingefahrenen Position. Bei Drehung des Implantatgrundkörpers 10 gegen das zweite Sicherungselement 70 in die zweite Position in Rotationsrichtung R wird das Befestigungselement 73 um 90° derart gedreht, dass seine Längsachse im wesentlichen parallel zu der in den Figuren 11 und 12 eingezeichneten y-Achse verläuft. Die Endposition ist in Figur 12 dargestellt. Dabei werden die Schwenkelemente 75a, 75b radial nach außen ausgefahren. Auf der Seite 70b des zweiten Sicherungselements 70 sind vier Anschläge 77a, 77b, 77c, 77d derart angeordnet, dass sie die Bewegung der Schwenkelemente 75a, 75b führen. Insbesondere wird das Schwenkelement 75a zwischen den beiden Vorsprüngen 77c, 77d und das Schwenkelement 75b zwischen den beiden Vorsprüngen 77a, 77b geführt. Der Abstand der Vorsprünge 77a, 77b bzw. 77c, 77d ist dabei so bemessen, dass in der ausgefahrenen Position der Schwenkelemente 75a, 75b die Schwenkelemente 75a, 75b zwischen den Vorsprüngen 77a, 77b bzw. 77c, 77d eingekeilt werden und somit gegen ein weiteres Verschwenken gegenüber dem Befestigungselement 73 gesichert sind.

Die beiden Schwenkelemente 75a, 75b sichern zusätzlich zu dem Sicherungselement 70 das Implantat dagegen, dass es durch den Zwischenraum zwischen zwei benachbarten Dornfortsätzen herausgedrückt werden kann, und weisen eine derartige anatomisch angepasste Form auf, dass sie sich ebenfalls wie die Vorsprünge 55a, 55b des zweiten Sicherungselements 50 gemäß der ersten Ausführungsform an die anatomischen Strukturen des Patienten anlegen und dabei eine Sicherung gegen Rotation des zweiten Sicherungselements 70 um die Längsachse 1 bieten.

Der Arretiermechanismus zur Sicherung des Implantatgrundkörpers 10 in der zweiten Position relativ zu dem zweiten Sicherungselement 70 und somit zur Sicherung der Schwenkelemente 75a, 75b in der ausgefahrenen Position wird durch ein drehfest an dem Befestigungselement 73 angeordnetes Rastelement 72 gebildet, welches mit zwei Zapfen 71 zusammenwirkt. Die Zapfen 71 sind radial nach außen auf einer Geraden auf der Seite 70b des zweiten Sicherungselements 70 angeordnet, wobei ihre Längsachse im wesentlichen parallel zur Längsachse 1 des Implantats 1 verläuft. Das Rastelement 72 ist etwa S-förmig ausgebildet und derart dimensioniert, dass jeweils einer der Bögen des S-förmigen Rastelements 72 einen der Zapfen zumindest teilweise umschließen kann. Die Bögen des S-förmigen Rastelements 72 können einen derartigen Winkelbereich umschließen, dass sie rastend die Zapfen 71 umschnappen und den Implantatgrundkörper 10 in der zweiten Position und die Schwenkelemente 75a, 75b in der ausgefahrenen Position fixieren. Zudem sind die Bögen derart ausgeformt, dass sie bei Drehung um etwa 90° gegen die Rotationsrichtung R bereits mit ihrer Außenfläche an die zapfen 71 anschlagen, so dass auf diese Weise die Drehbewegung des Implantatgrundkörpers relativ zu dem zweiten Sicherungselement auf einen Winkelbereich von etwa 90° beschränkt wird.

Der Implantatgrundkörper 10 weist zusätzlich im Gegensatz zu dem Implantatgrundkörper 10 des ersten Ausführungsbeispiels ausgehend von der Längsbohrung 33 oder auch ausgehend von der Bohrung 38, insgesamt aber im wesentlichen ausgehend von der Längsachse 1 des Implantatgrundkörpers 10 eine Nut 33a auf, welche sich über die Außenseite des ersten Sicherungselements 20 bis zu dem Vorsprung 31b erstreckt (vgl. Fig. 13 und 14). Wenn eine Einführhilfe wie beispielsweise ein Draht oder ähnliches durch das Implantat 1 geführt wird, kann der Draht beim Einführen des Implantats 1 in den Körper des Patienten in der Nut 33a auf der Außenseite des Implantats 1 geführt werden.

### Bezugszeichenliste

- 1: Dornfortsatzimplantat

- 10: Implantatgrundkörper
- 12: Zapfen
- 14: Sackbohrung

- 20: erstes Sicherungselement

- 30: Spreizelement
- 31: Anlagefläche
- 31a: Vorsprung
- 31b: Vorsprung
- 32: Spitze
- 33: Längsbohrung
- 33a: Nut
- 37: Schraube
- 38: Bohrung

- 40: Rastwelle
- 42: unrunder Abschnitt
- 43: Anlagefläche
- 44: Eingriffsbohrung
- 46: Längsbohrung

- 50: zweites Sicherungselement
- 50a: Seite
- 50b: Seite
- 51: Aufnahme
- 51a: Führung
- 51b: seitlicher Schlitz
- 53: Nut
- 54: Rastausnehmung
- 55: Anlagefläche
- 55a: Vorsprung
- 55b: Vorsprung
- 56: Öffnung

- 60: Splint
- 62: Ausnehmung
- 62a: erster Bereich
- 62b: zweiter Bereich
- 64: Rastnase

- 70: zweites Sicherungselement
- 70a: Seite
- 71: Zapfen
- 72: Rastelement
- 73: Befestigungselement
- 75a: Schwenkelement
- 75b: Schwenkelement
- 76: Öffnung
- 77a: Vorsprung
- 77b: Vorsprung
- 77c: Vorsprung
- 77c: Vorsprung

- 90: Wirbelkörper
- 91: Wirbelkörper
- 95: Dornfortsatz
- 96: Dornfortsatz

- 1: Längsachse
- W: Achse der Wirbelsäule
- h: Hauptachse

- n: Nebenachse

- x: x-Achse
- y: y-Achse

- R: Rotationsrichtung

## Patentansprüche

1. Dornfortsatzimplantat (1) mit einem Implantatgrundkörper (10) zum Einbringen zwischen Dornfortsätze (95, 96), an welchem ein erstes Sicherungselement (20) und ein zweites Sicherungselement (50) derart angeordnet sind, dass die Sicherungselemente (20, 50) nach Einbringen des Implantatgrundkörpers (10) zwischen die Dornfortsätze (95, 96) auf verschiedenen Seiten der Dornfortsätze (95, 96) zu liegen kommen, wobei das erste Sicherungselement (20) relativ zu dem Implantatgrundkörper (10) fixiert ist und wobei der Implantatgrundkörper (10) relativ zu dem zweiten Sicherungselement (50) um eine Längsachse (1) zwischen einer ersten Position und einer zweite Position verdrehbar an dem zweiten Sicherungselement (50) angeordnet ist, wobei ein Arretiermechanismus zur Fixierung des Implantatgrundkörpers (10) in der zweiten Position vorgesehen ist,
wobei der Arretiermechanismus durch einen Splint (60) gebildet ist, **dadurch gekennzeichnet, dass** der Splint (60) zwischen einer ersten Position und einer zweiten Position verschiebbar ist und von einer Rastwelle (40) des Implantatgrundkörpers (10) durchsetzt wird, welche einen unrunden Abschnitt (42) aufweist, wobei in der ersten Position die Rastwelle (40) um ihre Längsachse in einer Ausnehmung (62) des Splints (60) drehbar ist, und wobei in der zweiten Position die Ausnehmung (62) einen Formschluss mit dem unrunden Abschnitt (42) der Rastwelle (40) bildet, wodurch eine Drehung der Rastwelle (42) verhindert wird.

2. Dornfortsatzimplantat nach Anspruch 1,
**dadurch gekennzeichnet, dass** der Arretiermechanismus ein Rastmechanismus ist.

3. Dornfortsatzimplantat nach Anspruch 1 oder 2,
**dadurch gekennzeichnet, dass** der Splint (60) in einer Aufnahme (51) in dem zweiten Sicherungselement (50) geführt ist.

4. Dornfortsatzimplantat nach einem der Ansprüche 1 bis 3,
**dadurch gekennzeichnet, dass** der Splint (60) wenigstens eine Rastnase (64) aufweist, welche in der zweiten Position des Implantatgrundkörpers (10) mit einer Rastausnehmung (54) in Eingriff bringbar ist.

5. Dornfortsatzimplantat nach einem der vorhergehenden Ansprüche,
**dadurch gekennzeichnet, dass** an dem Implantatgrundkörper (10) ein Vorsprung angeordnet ist, welcher mit zwei an dem zweiten Sicherungselement (50) angeordneten Vorsprüngen derart zusammenwirkt, dass die Drehbewegung des Implantatgrundkörpers (10) relativ zu dem zweiten Sicherungselement (50) auf einen bestimmten Winkelbereich begrenzt wird.

6. Dornfortsatzimplantat nach Anspruch 5,
**dadurch gekennzeichnet, dass** der an dem Implantatgrundkörper angeordnete Vorsprung an wenigstens einem der an dem zweiten Sicherungselement (70) angeordneten Vorsprüngen über einen Rastmechanismus fixierbar ist, welcher vorzugsweise den Arretiermechanismus bildet.

7. Dornfortsatzimplantat nach Anspruch 5 oder 6,
**dadurch gekennzeichnet, dass** der an dem Implantatgrundkörper angeordnete Vorsprung als Rastelement (72) und die an dem Sicherungselement angeordnetten Vorsprünge als Zapfen (71) ausgebildet sind.

8. Dornfortsatzimplantat nach einem der vorhergehenden Ansprüche,
**dadurch gekennzeichnet, dass** an dem Implantatgrundkörper (10) ein in Längsrichtung vorstehender, exzentrisch angeordneter Zapfen (12) angeordnet ist, welcher in eine kreisbogenförmige Nut (53) des zweiten Sicherungselements (50) eingreift, wobei die Nut (53) vorzugsweise einen Winkelbereich von etwa 90° überstreicht.

9. Dornfortsatzimplantat nach einem der vorhergehenden Ansprüche,
**dadurch gekennzeichnet, dass** das erste Sicherungselement (20) einstückig mit dem Implantatgrundkörper (10) zu einem Spreizelement (30) verbunden ist.

10. Dornfortsatzimplantat nach Anspruch 9,
**dadurch gekennzeichnet, dass** das Spreizelement (30) aus einem Kunststoff oder einem biokompatiblen Material mit hohem Elastizitätsmodul, insbesondere aus Polyetheretherketon, gefertigt ist.

11. Dornfortsatzimplantat nach Anspruch 9 oder 10,
**dadurch gekennzeichnet, dass** das Spreizelement (30) eine anatomisch geformte Anlagefläche (31) aufweist, insbesondere zur Anlage an die benachbarten Dornfortsätze (95, 96).

12. Dornfortsatzimplantat nach einem der vorhergehenden Ansprüche,
**dadurch gekennzeichnet, dass** das erste Sicherungselement (20) eine anatomisch geformte Spitze (32) zur Durchführung durch den Zwischenraum zwischen den Dornfortsätzen (95, 96) aufweist.

13. Dornfortsatzimplantat nach einem der vorhergehenden Ansprüche,
**dadurch gekennzeichnet, dass** das zweite Sicherungselement (50, 70) eine anatomisch geformte Anlagefläche (55) aufweist, insbesondere zur Anlage an dem Lamina und an dem Fassettengelenk.

14. Dornfortsatzimplantat nach einem der vorhergehenden Ansprüche,
**dadurch gekennzeichnet, dass** das zweite Sicherungselement (70) mindestens ein, vorzugsweise zwei bis vier Schwenkelemente (75a, 75b) aufweist, welche relativ zu dem zweiten Sicherungselement (70) bewegbar sind.

15. Dornfortsatzimplantat nach Anspruch 14,
**dadurch gekennzeichnet, dass** die Schwenkelemente (75a, 75b) zwischen einer eingefahrenen Position und einer ausgefahrenen Position schwenkbar sind, wobei sich die Schwenkelemente (75a, 75b) bevorzugt in der eingefahrenen Position befinden, wenn sich der Implantatgrundkörper (10) in der ersten Position befindet, und sich in der ausgefahrenen Position befinden, wenn sich der Implantatgrundkörper (10) in der zweiten Position befindet.

16. Dornfortsatzimplantat nach einem der vorhergehenden Ansprüche,
**dadurch gekennzeichnet, dass** das Dornfortsatzimplantat (1) eine Längsbohrung (32) mit einem Einstich aufweist.

17. Dornfortsatzimplantat nach einem der vorhergehenden Ansprüche,
**dadurch gekennzeichnet, dass** das Dornfortsatzimplantat (1) kannuliert ist.

18. Dornfortsatzimplantat nach einem der vorhergehenden Ansprüche,
**dadurch gekennzeichnet, dass** in dem ersten Sicherungselement (20) ausgehend etwa von der Längsachse (1) eine Nut (33a) angeordnet ist.

19. Dornfortsatzimplantat nach einem der vorhergehenden Ansprüche,
**dadurch gekennzeichnet, dass** der Implantatgrundkörper (10) einen im wesentlichen ovalen Querschnitt mit einer Hauptachse (h) und einer Nebenachse (n) aufweist, wobei vorzugsweise die Hauptachse (h) in der zweiten Position etwa parallel zur Wirbelsäule verläuft.

20. Dornfortsatzimplantat nach Anspruch 19,
**dadurch gekennzeichnet, dass** die Hauptachse (h) etwa 2 mm länger ist als die Nebenachse (n).

## Claims

1. Spinous process implant (1) comprising an implant main body (10) for insertion between spinal processes (95, 96), on which a first securing element (20) and a second securing element (50) are arranged in such a way that the securing elements (20, 50) come to lie on different sides of the spinous processes (95, 96) after the implant main body (10) has been inserted between the spinous processes (95, 96), wherein the first securing element (20) is fixed relative to the implant main body (10) and wherein the implant main body (10) is arranged on the second securing element (50) in such a way as to be able to rotate about a longitudinal axis (1) relative to the second securing element (50) between a first position and a second position, wherein an immobilising mechanism is provided for fixing the implant main body (10) in the second position, wherein the immobilising mechanism is formed by a splint (60), **characterised in that** the splint (60) is displaceable between a first position and a second position and is penetrated by a latching shaft (40) of the implant main body (10) which has a non-circular section (42), wherein in the first position the latching shaft (40) is rotatable about its longitudinal axis in a cutout (62) of the splint (60), and wherein in the second position the cutout (62) establishes a form fit with the non-circular section (42) of the latching shaft (40), thereby preventing rotation of the latching shaft (42).

2. Spinous process implant according to claim 1, **characterised in that** the immobilising mechanism is a latching mechanism.

3. Spinous process implant according to claim 1 or 2, **characterised in that** the splint (60) is guided in a slot (51) in the second securing element (50).

4. Spinous process implant according to one of claims 1 to 3, **characterised in that** the splint (60) has at least one latching protrusion (64) which in the second position of the implant main body (10) can be brought into engagement with a latching recess (54).

5. Spinous process implant according to one of the preceding claims, **characterised in that** there is arranged on the implant main body (10) a protrusion which cooperates with two protrusions arranged on the second securing element (50) in such a way that the rotational movement of the implant main body (10) relative to the second securing element (50) is limited to a certain angular range.

6. Spinous process implant according to claim 5, **characterised in that** the protrusion arranged on the implant main body can be fixed to at least one of the protrusions arranged on the second securing element (70) via a latching mechanism which preferably forms the immobilising mechanism.

7. Spinous process implant according to claim 5 or 6, **characterised in that** the protrusion arranged on the implant main body is designed as a latching element (72) and the protrusions arranged on the securing element are designed as pins (71).

8. Spinous process implant according to one of the preceding claims, **characterised in that** there is arranged on the implant main body (10) an eccentrically arranged pin (12) which protrudes in the longitudinal direction and which engages in a circular arc-shaped groove (53) of the second securing element (50), wherein the groove (53) preferably covers an angular range of approximately 90°.

9. Spinous process implant according to one of the preceding claims, **characterised in that** the first securing element (20) is connected in one piece with the implant main body (10) to form a spreading element (30).

10. Spinous process implant according to claim 9, **characterised in that** the spreading element (30) is made from a plastic or a biocompatible material with a high modulus of elasticity, in particular from polyether ether ketone.

11. Spinous process implant according to claim 9 or 10, **characterised in that** the spreading element (30) has an anatomically shaped bearing surface (31), in particular for bearing against the adjacent spinous processes (95, 96).

12. Spinous process implant according to one of the preceding claims, **characterised in that** the first securing element (20) has an anatomically shaped tip (32) for passing through the intermediate space between the spinous processes (95, 96).

13. Spinous process implant according to one of the preceding claims, **characterised in that** the second securing element (50, 70) has an anatomically shaped bearing surface (55), in particular for bearing against the lamina and against the facet joint.

14. Spinous process implant according to one of the preceding claims, **characterised in that** the second securing element (70) comprises at least one, preferably two to four, pivoting elements (75a, 75b) which are movable relative to the second securing element (70).

15. Spinous process implant according to claim 14, **characterised in that** the pivoting elements (75a, 75b) are pivotable between a retracted position and an extended position, wherein the pivoting elements (75a, 75b) are preferably in the retracted position when the implant main body (10) is in the first position, and are in the extended position when the implant main body (10) is in the second position.

16. Spinous process implant according to one of the preceding claims, **characterised in that** the spinous process implant (1) has a longitudinal bore (32) with a puncture hole.

17. Spinous process implant according to one of the preceding claims, **characterised in that** the spinous process implant (1) is cannulated.

18. Spinous process implant according to one of the preceding claims, **characterised in that** a groove (33a) is arranged in the first securing element (20), starting approximately from the longitudinal axis (1).

19. Spinous process implant according to one of the preceding claims, **characterised in that** the implant main body (10) has a substantially oval cross-section with a main axis (h) and an auxiliary axis (n), wherein preferably the main axis (h) in the second position runs approximately parallel to the spinal column.

20. Spinous process implant according to claim 19, **characterised in that** the main axis (h) is approximately 2 mm longer than the auxiliary axis (n).

## Revendications

1. Implant pour processus épineux (1) comportant un corps de base d'implant (10) pour être installé entre les processus épineux (95, 96) que l'on a associé au premier élément de fixation (20) et un second élément de fixation (50) de façon que ces éléments de fixation (20, 50) après mise en place du corps de base de l'implant (10) entre les processus épineux (95, 96), arrivent en appui sur différents côtés des processus épineux (95, 96),
le premier élément de fixation (20) étant bloqué par rapport au corps de base de l'implant (10), et
le corps de base de l'implant (10) est installé par rapport au second élément de fixation (50) autour d'un axe longitudinal (1) de façon à pouvoir tourner entre une première position et une seconde position sur le second élément de fixation (50),
un mécanisme de blocage étant prévu dans la seconde position pour fixer le corps de base de l'implant (10),
le mécanisme de blocage étant constitué par une clavette (60),
**caractérisé en ce que**
la clavette (60) peut coulisser entre une première position et une seconde position, et elle est traversée par un axe d'enclipage (40) du corps de base d'implant (10), ayant un segment non circulaire (42), et dans la première position, l'axe d'enclipage (40) peut tourner autour de son axe longitudinal dans une cavité (62) de la clavette (60), et dans la seconde position, la cavité (62) réalise une liaison par la forme avec le segment non circulaire (42) de l'axe d'enclipage (40) pour interdire la rotation de l'axe d'enclipage (40).

2. Implant pour processus épineux selon la revendication 1,
**caractérisé en ce que**
le mécanisme de blocage est un mécanisme à enclipage.

3. Implant pour processus épineux selon la revendication 1 ou 2,
**caractérisé en ce que**
la clavette (60) est guidée dans un logement (51) du second élément de fixation (50).

4. Implant pour processus épineux selon l'une des revendications 1 à 3,
**caractérisé en ce que**
la clavette (60) comporte au moins un bec d'enclipage (64) mis en prise avec une cavité d'enclipage (54) dans la seconde position du corps de base d'implant (10).

5. Implant pour processus épineux selon l'une des revendications précédentes,
**caractérisé en ce que**
le corps de base d'implant (10) comporte une partie en saillie coopérant avec deux parties en saillie prévues sur le second élément de fixation (50) pour limiter le mouvement de rotation du corps de base d'implant (10) par rapport au second élément de fixation (50) à une certaine plage angulaire.

6. Implant pour processus épineux selon la revendication 5,
**caractérisé en ce que**
la partie en saillie prévue sur le corps de base d'implant peut être fixée à au moins l'une des parties en saillie prévue sur le second élément de fixation (70) par un mécanisme d'enclipage qui constitue de préférence le mécanisme de blocage.

7. Implant pour processus épineux selon la revendication 5 ou 6,
**caractérisé en ce que**
la partie en saillie prévue sur le corps de base d'implant est réalisée sous forme d'un élément d'enclipage (72) et les parties en saillie prévues sur l'élément de fixation, sont des tétons (71).

8. Implant pour processus épineux selon l'une des revendications précédentes,
**caractérisé en ce que**
le corps de base d'implant (10) comporte un téton (12) excentré, venant en saillie dans la direction longitudinale et pénétrant dans une rainure (53) en arc de cercle dans le second élément de fixation (50), la rainure (53) couvrant de préférence une plage angulaire d'environ 90°.

9. Implant pour processus épineux selon l'une des revendications précédentes,
**caractérisé en ce que**
le premier élément de fixation (20) est relié en une seule pièce au corps de base (10) de l'implant pour former l'élément d'écartement (30).

10. Implant pour processus épineux selon la revendication 9,
**caractérisé en ce que**
l'élément d'écartement (30) est en une matière plastique ou une matière biocompatible à module d'élasticité élevé, notamment en polyéther éther cétone.

11. Implant pour processus épineux selon la revendication 9 ou 10,
**caractérisé en ce que**
l'élément d'écartement (30) comporte une surface d'appui (31) de forme anatomique, notamment pour s'appuyer contre les processus épineux voisins (95, 96).

12. Implant pour processus épineux selon l'une des revendications précédentes,
**caractérisé en ce que**
le premier élément de fixation (20) a une pointe (32) de forme anatomique pour le passage dans l'intervalle entre les processus épineux (95, 96).

13. Implant pour processus spinosus selon l'une des revendications précédentes,
**caractérisé en ce que**
le second élément de fixation (50, 70) a une surface d'appui (55) de forme anatomique, notamment pour s'appuyer contre le lamina et l'articulation des facettes.

14. Implant pour processus spinosus selon l'une des revendications précédentes,
**caractérisé en ce que**
le second élément de fixation (70) comporte au moins un et de préférence entre deux et quatre éléments de pivotement (75a, 75b) qui sont mobiles par rapport au second élément de fixation (70).

15. Implant pour processus spinosus selon la revendication 14,
**caractérisé en ce que**
les éléments de pivotement (75a, 75b) peuvent pivoter entre une position rentrée et une position sortie,
les éléments de pivotement (75a, 75b) se trouvent de préférence en position rentrée lorsque le corps de base d'implant (10) se trouve dans sa première position et les éléments se trouvent dans la position sortie, lorsque le corps de base d'implant (10) occupe sa seconde position.

16. Implant pour processus spinosus selon l'une des revendications précédentes,
**caractérisé en ce que**
l'implant de processus épineux (1) comporte un perçage longitudinal (32) avec une entaille.

17. Implant pour processus spinosus selon l'une des revendications précédentes,
**caractérisé en ce que**
l'implant de processus épineux (1) est à canule.

18. Implant pour processus spinosus selon l'une des revendications précédentes,
**caractérisé en ce que**
partant de son axe longitudinal (1), le premier élément de fixation (20) comporte une rainure (33a).

19. Implant pour processus spinosus selon l'une des revendications précédentes,
**caractérisé en ce que**
le corps de base d'implant (10) a une section essentiellement ovale, l'axe principal (h) et l'axe auxiliaire (n), de préférence l'axe principal (h) passant dans la seconde position sensiblement parallèlement à la colonne vertébrale.

20. Implant pour processus spinosus selon la revendication 19,
**caractérisé en ce que**
l'axe principal (h) est plus long sensiblement de 2 mm que l'axe auxiliaire (n).
